# EUROPEAN PATENT SPECIFICATION

(11) **EP 1 803 392 B1**
(45) Date of publication and mention of the grant of the patent: **19.08.2009**
(21) Application number: 06126333.1
(22) Date of filing: 18.12.2006
(51) Int. Cl.: A61B 5/0432, A61B 5/0404

(54) **ECG recording device and method of use**
EKG-Aufnahmegerät und Verwendungsverfahren
Dispositif d'enregistrement d'un ECG et méthode d'utilisation

(30) Priority: 28.12.2005 US 319640
(43) Date of publication of application: 04.07.2007
(73) Proprietor: GENERAL ELECTRIC COMPANY, Schenectady, NY 12345 (US)
(72) Inventor: KOHLS, Mark R., New Berlin, WI 53151 (US)
(74) Representative: Illingworth-Law, William Illingworth

(56) References cited:
- WO-A-94/01040
- US-A- 41 804
- US-A1- 2002 115 912

## Description

This invention relates generally to the field of diagnostic cardiology, and more particularly, pertains to a portable, non-invasive recording apparatus capable of collecting information from a patient related to the patient's electrocardiogram (ECG) data at a location remote from a physician's office, clinic or hospital.

Syncope (sudden loss of consciousness) and arrhythmias (abnormal rhythm) resulting from electrical instability within the heart are particularly problematical for diagnostic physicians to observe in living patients. These events can be of short duration and sudden onset, and may occur very infrequently with little warning. Early diagnosis of arrhythmias is very important because the longer arrhythmia continues without detection or treatment, the greater the chances of severe permanent damage resulting in possible heart failure and stroke. Several arrhythmia-related diseases including Long QT Syndrome (LQTS), Wolff-Parkinson-White Syndrome, and Brugada Syndrome are genetic and LQTS can also be acquired through the use of certain pharmaceuticals. A clinically important method for diagnosing arrhythmia problems involves the use of electrocardiograms (ECGs) for monitoring the electrical activity in one's heart. Typically, electric leads are placed on the body at specific locations and the electrical activity resulting from heart depolarization and polarization is recorded by each lead. During a cardiac cycle, the electrocardiogram produces a distinctive waveform comprised of a P-wave, a QRS complex and a T-wave which are carefully analyzed to form diagnoses and assess the efficacy of treatments such as drug therapy.

ECG recorders are generally designed for portable collection of electrocardiograph data from a patient once an initial ECG is taken at a physician's office, clinic or other health facility having an on-site, non-ambulatory electrocardiograph. Recordings from these portable devices are subsequently used to detect abnormalities in the heart's electrical activity caused by a patient's routine daily activities or heightened emotional or physical states. Such recording devices are constructed of two types. The first is a retrospective type recording system that analyzes collected data after completion of the collection phase. The second type is a real-time system which analyzes data as it is recorded. In either type, the ECG signals are typically received through a plurality of leads attached between electrodes running between various points on a patient's body and an associated unit worn about the patient's neck, waist or wrist. The majority of these portable ambulatory monitors are bulky and interfere with the patient's normal life. Patient compliance cannot always be relied upon and is a common problem in their use. Problems with body-worn, lead-type portable monitors also include inability to abide attendant skin irritation. Removal may be required for showering, bathing or other water exposure. Results from such lead-type dependent monitors may vary depending on the placement of the various multiple electrodes upon the body of the patient.

Recently portable, non-ambulatory ECG monitoring and recording devices have become available which operate on tactile-sensing from thumbs or hands of patients without necessarily requiring leads and electrodes to be placed on the body. These recent portable, non-ambulatory devices are designed to be retained by the patient as long term, continuous use monitors/recorders which display real-time results and transfer collected data to another location. Such ECG result-displaying designs, while offering advantages over the prior art tend to be expensive to produce and maintain and, accordingly are not intended for large scale use. It is also important to consider a more basic type of portable device which does not require a display of real-time results, is more affordable, is designed for limited use, is returnable for analysis of the collected ECG data and is disposable.

One scenario which exemplifies the need for an improved, portable, non-ambulatory, tactile-sensing ECG monitor and recorder of the type that is inexpensive and readily in demand for a large group of patients relates to the dispensing of new pharmaceuticals. As is well known, new drugs follow a rigorous evaluation process from compound discovery to final approval requiring many years of trials and many millions of dollars in investment. Typically, a new drug is evaluated with progressive screening throughout Phase I, II and III clinical trials. Even after Phase III approval of the drug, liability concerns dictate a desire and/or requirement to perform a Phase IV trial, also known as post-market surveillance. These Phase IV trials are necessary because even if the drug showed few complications during Phase III, there is still no guarantee that when dispensed to a large number of patients that problems will not be discovered. A critical component of this drug follow-up is the recording of an ECG, primarily to look for the existence of drug-induced long QT syndrome (LQTS). Long QT syndrome causes an abnormality of the heart's electrical system predisposing certain affected persons to an extremely fast heart rhythm which can lead to sudden loss of consciousness and may cause sudden death. The name of the long QT syndrome refers to the QT interval measured on the ECG. To perform the test today requires a patient taking a medication to return to a physician's office, clinic or other facility with a substantially large, stand-alone electrocardiograph. This can be very time consuming as well as expensive and impractical to do with a drug monitored patient population that may be in the tens or even hundreds of thousands.

Besides LQTS, there is concern for monitoring congenital LQTS which is a genetic or inherited condition that can lead to fatal arrhythmia. These arrhythmias often occur in teenage children during physical exertion (e.g. playing a sport in school that involves swimming or running) and are frequently fatal. An improved portable ECG recorder is needed which would allow for a very inexpensive mass screening of athletes and other students to look for heart problems at an early age in a manner which is unavailable today.

Additionally, there exists today the problem in hospitals of infection control. Patients with severe infections, such as MRSA (methycillin-resistant Staphylococcus aureus), may require an ECG be acquired. Currently, a hospital electrocardiograph is used to make the recording. However, following the recording, the recording device must be disinfected before further use. This involves detailed cleaning using severe cleaning agents which can damage and eventually destroy the ECG leads. It is important to consider an alternative portable ECG design which will address the problem of infection control.

US 2002/115912 A1 discloses a vital sign box with a plurality of vital sensors measuring predetermined biological, chemical, or physical conditions of a living body; a camera taking a picture of a predetermined object; and a housing containing the plurality of vital sensors and the camera. WO 94/01040 A1 discloses an ambulatory heart monitoring apparatus provided with a slot for receiving an IC card. A user places electrodes on his body. Operation of a switch causes the apparatus to record ECG signals from the electrodes onto the IC card.

Accordingly, it is desirable to provide an improved easy-to-use, portable, non-ambulatory, tactile-sensing ECG recorder that can be produced inexpensively in large quantities, and is made available for large scale use outside a hospital, clinic or other ECG-equipped health facility. Such a portable ECG recorder should be capable of personal design for distribution to a patient picking up a particular prescription, as well as an anonymous design to be used for a generalized screening purpose. It is also desirable to provide a portable, lightweight ECG device which will read and store rather than actually display one or more ECG recordings, and which can be easily returned to a central lab for review and analysis. Ideally, such design is intended to have a limited use and be disposable once returned to a facility for interpretation of the collected ECG data. Additionally, the ECG recorder should be designed to be safely used in a high infection risk environment without the need to clean and disinfect.

An aspect of the present invention is directed to a portable limited use electrocardiogram (ECG) recording device used for diagnosing cardiac problems. The device is movable between a folded, closed position and an unfolded, ECG-recording operative position. The recording device includes a cover hingedly connected to a base. The cover and the base each have an inner face exposed to a patient when in the operative condition. The inner face of the cover is provided with operating instructions and a patient questionnaire. The inner face of the base is provided with a pair of spaced apart, tactile-sensing electrodes which are engageable with the patient's hands during an ECG recording. The inner face of the base further includes at least one indicator light for signaling a start and finish status of the ECG recording. A circuit card is removably retained inside a slot in the base. The circuit card is designed to be removed from the slot in order to analyze the patient's ECG data. The circuit card is electrically connected to the electrodes and the status indicator light. The circuit card includes a source of power, a processor and memory for receiving and storing ECG signals from the patient touching the electrodes. The recorded ECG signals are sent to a remote location for retroactively analyzing the stored ECG signals.

In the preferred embodiment, the inner face of the cover includes pictorial operating instructions which are universally understandable to any patient. The inner face of the cover further includes a holder for receiving and retaining a first card having detailed text operating instructions printed in a desired language, and a second card for providing health and demographic information from the patient. The inner face of the base is provided with pictorial information reminding the patient to maintain their hands on the electrodes during the ECG recording, and to complete the patient questionnaire following completion of the ECG recording. The circuit is preferably formed as a card which is retained within the base. The circuit further includes a battery, leads connected to the electrodes, a user interface and an acquisition interface, all interconnected with the processor and the memory. The circuit card may include an internal connector adapted to be connected to a reading system for reading the stored ECG signal. The stored ECG signal is designed to be read such as by a wireless reading system, if desired. The base may also include an input connector in communication with the circuit and is adapted to receive at least one supplementary lead secured to an electrode positioned on a body portion of the patient. Additionally, the electrodes may also be used as a means of communication. The base may also have an outer face provided with an additional pair of spaced apart, tactile-sensing electrodes engageable with thighs of the patient. The inner face of the base may be formed with an indentation defining a sealable blood well for holding a blood sample of the patient. Preferably the recording device is provided along with medication prescribed to the patient. In this case, the circuit is preprogrammed according to the particular prescribed medication. The outer face of the cover also includes a bar code identifying the particular patient. The outer face of the cover further includes pre-paid postage and preprinted mailing information for returning the device.

An aspect of the invention also contemplates a method of limited term monitoring of the patient for cardiac problems. The method includes the steps of providing the portable electrocardiogram (ECG) device according to the present invention; indicating a start status to the patient when the device is in the unfolded, operative position by means of the status indicator light to signify an ECG recording is required; touching the electrodes with hands of the patient; collecting and storing the ECG signal when the patient touches the electrode; indicating a finished status to the patient by means of the status indicator light to signify the ECG signal has been recorded and transferring the stored ECG signal to a remote location for analysis thereof.

The method further includes disposing the device following analysis of the stored ECG signal. In the method, the device can be further provided with additional pair of spaced apart electrodes engageable with thighs of the patient, and the step of touching the electrodes includes touching with the thighs. The method also includes the step of notifying the patient with the results of the stored ECG signal following return of the device to the remote location.

In another aspect of the invention, the method of limited term monitoring of a patient for cardiac problems is performed in conjunction with the taking of a particular prescribed medication, whereby the method includes the steps of providing the patient with the portable personalized electrocardiogram (ECG) device of the present invention at the time the prescribed medication is dispensed.

Various aspects and embodiments of the present invention will now be described in connection with the accompanying drawings, in which:
Figure 1 is a perspective view of an ECG recording device embodying the present invention, the device being shown in a folded, closed package;
Figure 2 is a top view of the recording device of Fig. 1 in an unfolded, operative condition;
Figure 3 is a bottom view of Fig. 2;
Figure 4 is a view of a feedback card to be completed and returned by the patient after an ECG reading has been taken;
Figure 5 is a view of a how-to-use card;
Figure 6 is a block diagram of a circuit used in a recording device.

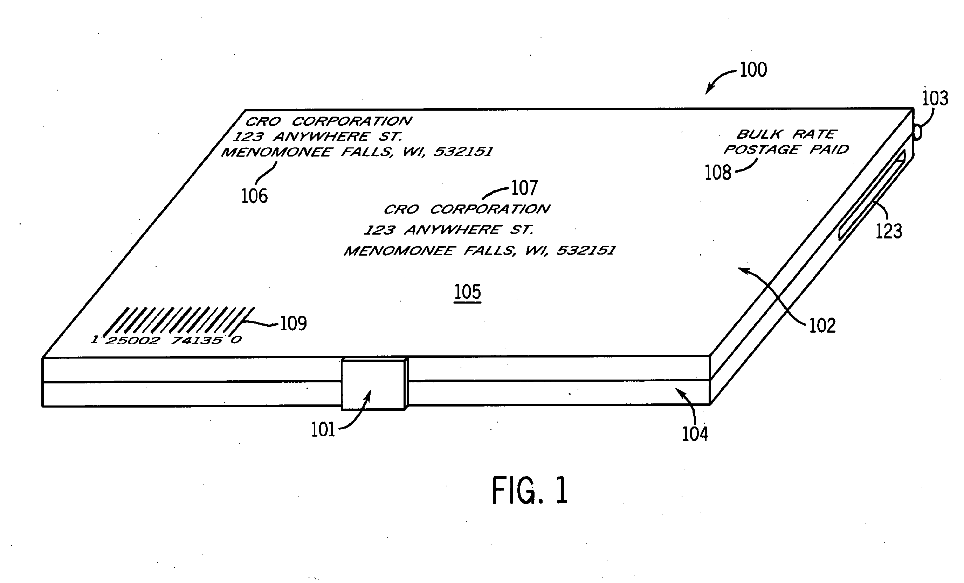

Referring now to the drawings, Fig. 1 illustrates a portable, non-ambulatory and non-invasive, electrocardiogram (ECG) recording device 100 used for monitoring and recording ECG waveforms by a patient preferably at a location remote from a physician's office, clinic or other ECG-equipped facility. The recording device 100 is dispensed, such as at a pharmacy or other outlet, in a package normally kept in a folded condition by any suitable closure member 101. The recording device 100 includes a planar cover 102 hingedly connected, such as by a spine 103, to a planar base 104. Both the cover 102 and the base 104 are constructed of a lightweight, rigid material, such as paperboard, foam or plastic, and are similarly sized and shaped. In the preferred embodiment, the cover 102 and the base 104 are both of rectangular shape with an exemplary width of 0.2032 m (8 inches) and a length of 0.2794 m (11 inches). In the folded condition, the thickness of the package is typically less than 0.0254 m (one inch).

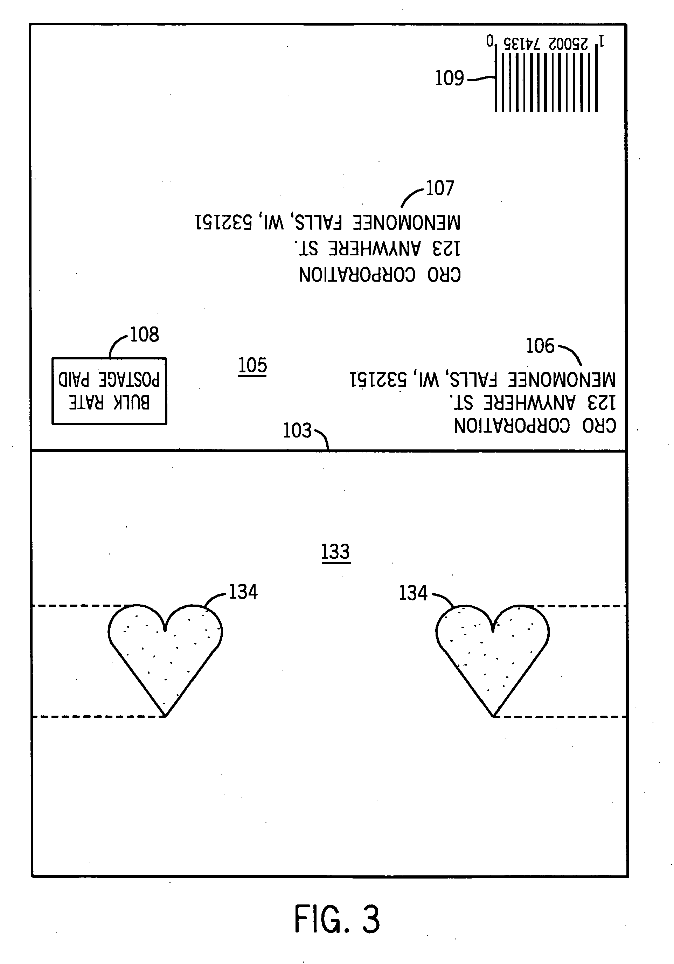

As will be further understood, the recording device 100 is designed as a self-mailer and the cover 102 has an outer face 105 provided with a return address 106, a mailing address 107, paid postage 108 and a bar code 109 which can be used to identify the patient receiving the device 100. Figure 3 illustrates the outer face 105 of cover 102 in more detail, and shows a bottom view of the recording device 100 once closure 101 has been opened and the device 100 is in an unfolded operative condition.

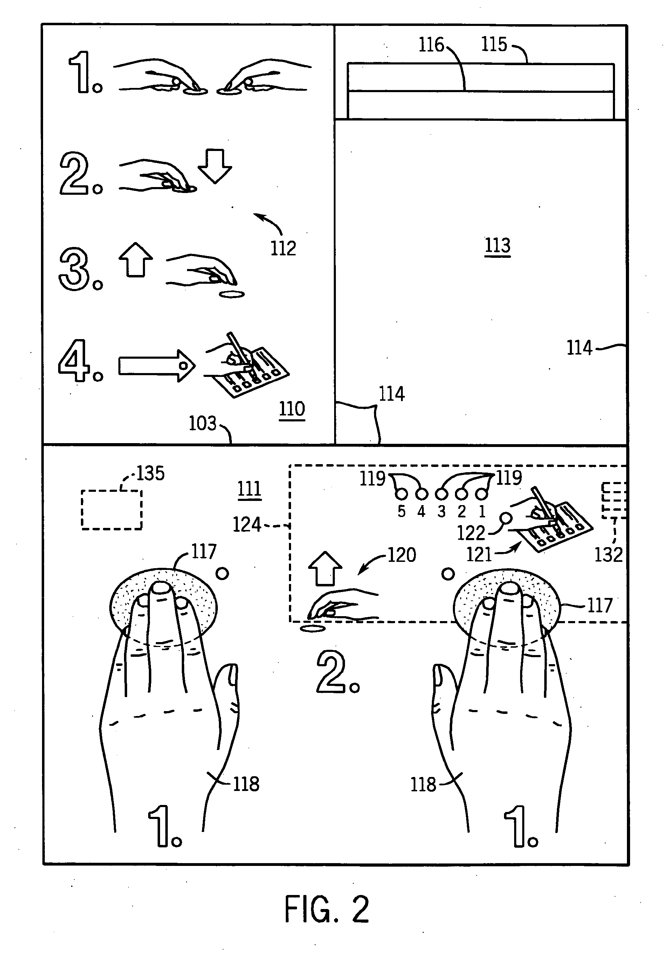

When the ECG recording device 100 is unfolded, as shown in Fig. 2, the patient views an inner face 110 of the folded back cover 102, and an inner face 111 of the base 104 which are divided by the spine 103.

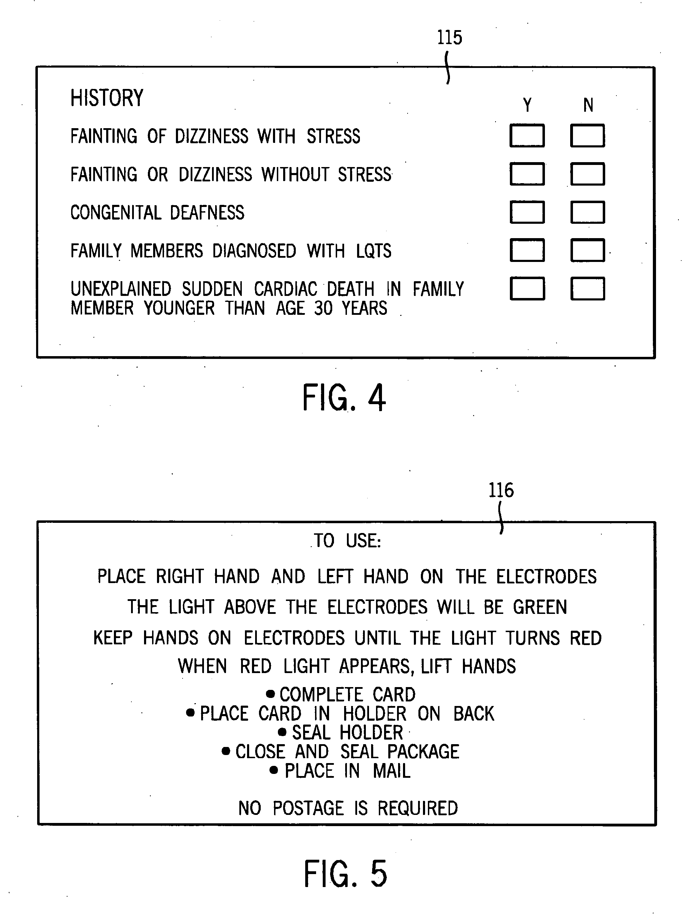

On one side, namely the left side, of inner face 110, a set of universally understandable pictorial instructions 112 is provided for guiding the patient in use of the device 100. On the opposite side, namely the right side of inner face 110 is a pocket or holder 113 generally defined by closed sides 114. The holder 113 is typically open at its top end to receive and retain a feedback card or questionnaire 115 and a more detailed instruction card 116 for operating device 100. The top end of holder 113 may be sealed. The feedback card 115 (Fig. 4) is particularly designed with a series of health and demographic questions for the patient to answer based on the medication prescribed to the patient with the dispensing of the recording device 100. The detailed instruction card 116 (Fig. 5) more precisely elaborates on the pictorial instructions 112, and may be written in a specific language for the particular patient.

The inner face 111 of base 104 is provided with a pair of spaced apart, tactile-sensing dry electrodes 117. The electrodes 117 are contacted by four fingers of the patient's right and left hands 118 during an ECG reading. At least one status indicator light 119 is included on the inner face 111 to provide a universally recognizable indication to the patient of the status of a particular ECG reading. In the example shown, there are several such indicator lights 119 to signify that multiple separate readings are required. In the preferred embodiment, each indicator light 119 changes color during a reading. For example, the indicator light 119 identified at "5" for the first of the readings, shows green indicating an ECG reading is ready to be taken. The patient's hands 118 are placed on the electrodes 117 and retained there until the light 119 turns to red indicating the termination of the first ECG reading. The lights 119 for the remaining ECG readings will operate identically and the patient will be instructed as to when to take the subsequent readings according to further information included on instruction card 116 and/or his/her particular instructions accompanying the drug prescription. A pictorial instruction 120 is provided on the inner face 111 between the electrodes 117 to remind the patient to hold their hands 118 upon the electrodes 117 until the particular indicator light 119 turns red at which time they may raise their hands 118 from the electrodes 117.

A further pictorial instruction 121 on inner face 111 may include a reminder light 122 to instruct the patient to complete the questionnaire or feedback card 115 following the completion of an ECG reading.

As best seen in Fig. 1, the interior of base 104 is formed with a slot 123 for receiving and retaining a recoverable circuit card 124 which provides power, the necessary interconnections to the electrodes 117 and lights 119, and sufficient memory to record the ECG data. The circuit card is programmed in the preferred embodiment for the specific patient and his/her drug therapy. The circuit card 124 is removable from the device 100 after the device is returned by mail or similar carrier to a specific location to have the patient's ECG reading(s) analyzed.

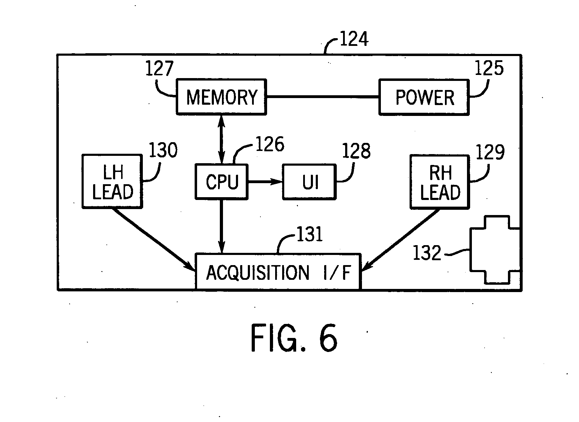

Figure 6 shows a block diagram of the circuit card 124 which includes a source of power 125, such as a battery, interconnected to a central processing unit (CPU) 126 having a memory 127 in communication with a user interface (UI) 128. Right and left hand leads 129, 130 are joined to the electrodes 117. All electrical data sent from the patient through the electrodes 117 is converted to digital information stored in memory 127 by an acquisition interface 131 in electrical communication with elements 125-130. The circuit card 124 may also be optionally provided with an internal connector 132 where a reading system can be connected to the device 100 once the device is returned to its central location for analysis. This connector 132 can further be utilized in a mode to program the number of desired ECG readings or download additional information such as demographics or tracking information. Ideally, this connector 132 can implement a technology such as "near field communication" so that it would not be necessary to require actual physical connection to the device 100. Instead, the collected ECG data could be read out, such as by an IF or RF device. For infection control concerns, the device 100 with collected ECG data can be sealed in a sterile plastic bag and then safely read by scanning the protected device 100 using the IF or RF device.

In an enhanced version of the device 100, an outer face 133 of base 104 includes an additional pair of spaced apart, dry tactile-sensing electrodes 134 which are intended to contact the tops of the patient's right and left thighs and supply additional readings when the device 100 rests upon the patient's lap. The electrodes 134 may extend laterally to the side edges of base 104 (as shown in dotted lines) so that the device 100 may be alternatively placed between the patient's thighs. In this version, the electrodes 134 would have additional leads coupled to the circuit card 124.

Further optional considerations include the provision of a small indentation 135 for a sealable blood well along with a small pin (not shown) to draw blood from a finger. Such feature would allow for genetic testing which is useful in diagnosing arrhythmic abnormalities. In addition, a further input connector 136 may be provided within base 104 to provide a further connector point to circuit card 124 where a cable with additional disposable leads (not shown) as provided could be attached if desired for attachment to the patient's body. More leads would allow for the screening of more types arrhythmic conditions, such as Brugada syndrome. In a more advanced application, the device 100 could be preprogrammed to beep when it is time to take an ECG reading based on a protocol of a study. This would require insertion in the base 104 of a small audio device, such as a buzzer or speaker.

Various aspects of the invention further contemplates transmission of the recorded ECG data back to the central receiving site without the need to mail back the device 100. Accordingly, further optional considerations include the provision of a speaker 137 on the base 104 that allows for an acoustically-coupled transmission. In this form, the user dials a phone number from his/her phone and places the phone mouthpiece over the speaker 137 so that recorded data is modulated at the normal voice frequency and decided at the central receiving site. In another version, it is envisioned adding a phone jack so that when the device 100 is plugged into the jack, it dials a preprogrammed phone number and enables a digital transmission back to the central site. Alternatively, a network connection can be added to permit a network transfer of ECG data over the Internet back to a preprogrammed central site. Furthermore, data could be optionally encrypted on the device 100 and during transmission of data.

In a preferred use of the basic device 100, a patient picking up a prescription, such as from a pharmacy, is concurrently giving the portable ECG recording device 100 which is preprogrammed to request a predetermined set of ECG readings at prescribed intervals based upon the patient's drug therapy. The device 100 is intended to be used by the patient at home or another location remote from a doctor's office, clinic or other facility with an electrocardiogram. Upon opening the device 100, the patient will remove and review the feedback and detailed use instruction cards 115, 116 from the holder 113. User instructions are pictorially summarized at 112 which are constantly visible to the patient. When ready to take an ECG, the patient positions the bottom of base 104 on his/her lap, and places the fingers of their hands 118 on the electrodes 117. The status indicator light(s) 119 will show green until the reading is finished and the light(s) will change to red. This prompts the patient to remove his/her hands 118 from the electrodes 117 and complete the feedback card 115. The completed card 115 is replaced in the holder 113. When any subsequent required recordings are completed, the entire recording device 100 is closed by closure member 101. The entire device 100 is then returned, such as by U.S. mail or the like, to a central location for analysis due to the self mailing design of the outer face 105 of cover 102. The patient can be automatically notified that the device 100 has been received. ECG data stored in the circuit card 124 is then read and analyzed, and the patient and the physician are advised of the results. Once the ECG data has been read, the entire device 100 is disposed of.

Alternatively, or in addition to, the system that reads the ECG data and programs the device 100 could also be programmed to call the patient on appropriate days to remind them to perform the ECG recording. In this option, the patient would need to register at a secure website. Registration at the website would also permit the patient to enter demographic and health information as well as a responses to the questions on the feedback card 115.

Device 100 having additional leads or other modifications would have an operational card 116 explaining their modifications.

While the ECG recording device 100 has been described as being used with prescribed medication, it should be understood that the device due to its affordability may be sold in retail stores to allow individual consumers to acquire ECGs that could be used to screen for other problems such as caused by allergies. The device 100 can be provided in schools and communities to enable screening, and may be useful in insurance evaluations and other applications.

Various embodiments of the present invention thus provide a portable, non-ambulatory, non-invasive, disposable ECG recording device 100 which can be specifically designed to be given to and operated in a simple matter by a patient under a prescribed drug therapy. The device is designed to be produced in great quantities for a large scale use at a very low cost, is intended to read out data retrospectively, and can be branded or left anonymous. The packaging of the device is integrated for shipment back to a central location for analyzing the ECG recordings and reporting to the patient. The device can be used in a high risk environment safely without the need to clean and disinfect.

Various alternatives and embodiments are contemplated as being within the scope of the following claims defining the invention.

### PARTS LIST

| Part # | Part name | | Part # | Part Name |
|---|---|---|---|---|
| 100 | ECG recording device | | 124 | circuit card |
| 101 | closure member | | 125 | source of power |
| 102 | cover | | 126 | CPU |
| 103 | spine | | 127 | memory |
| 104 | base | | 128 | user interface |
| 105 | outer face of cover | | 129 | right hand lead |
| 106 | return address | | 130 | left hand lead |
| 107 | mailing address | | 131 | acquisition interface |
| 108 | paid postage | | 132 | internal connector |
| 109 | bar code | | 133 | outer face of base |
| 110 | inner face of cover | | 134 | thigh electrodes |
| 111 | inner face of base | | 135 | indentation |
| 112 | pictorial instructions | | 136 | input connector |
| 113 | holder | | | |
| 114 | closed sides | | | |
| 115 | feedback card | | | |
| 116 | detailed instruction card | | | |
| 117 | electrodes | | | |
| 118 | hands | | | |
| 119 | status indicator light | | | |
| 120 | pictorial instruction | | | |
| 121 | pictorial instruction | | | |
| 122 | reminder light | | | |

## Claims

1. A portable, limited use electrocardiogram (ECG) recording device (100) for diagnosing cardiac problems, the device (100) being movable between a folded, closed position and unfolded, ECG recording operative position, the device (100) comprising:
a cover (102) hingedly connected to a base (104), the cover (102) and the base (104) each having an inner face (110, 111) exposed to a patient when in the operative condition, the inner face (110) of the cover (104) being provided with operating instructions (116) and a patient questionnaire (115), the inner face (111) of the base (104) being provided with a pair of spaced apart, tactile-sensing electrodes (117) engageable with the patient's hands (118) during an ECG recording and at least one indicator light (119) for signaling a start and finish status of the ECG recording; and
means (107, 137) transferring the stored ECG signals to a remote location for analysis thereof, **characterised by**
a circuit card (124) removably retained inside a slot (123) in the base (104), the circuit card being designed to be removed from the slot (123) in order to analyze the patient's ECG data; the circuit card (124) being electrically connected to the electrodes (117) and the status indicator light (119), the circuit card (124) including a source of power (125), a processor (126) and memory (127) for receiving and storing ECG signals from the patient touching the electrodes (117).

2. The device of claim 1, wherein the cover (102) has an outer face (105) provided with preprinted mailing information (107) for returning the recording device (100) in the folded, closed position to the location for retroactively analyzing the stored ECG signals.

3. The device of claim 1 or claim 2, wherein the inner face (110) of the cover (102) includes pictorial operating instructions (112) which are universally understandable to any patient.

4. The device of any preceding claim, wherein the inner face (110) of cover (102) further includes a holder (113) for receiving and retaining a first card (116) having detailed text operating instructions printed in a desired language, and a second card (115) for providing health and demographic information from the patient.

5. The device of any preceding claim, wherein the inner face (111) of the base (104) is provided with pictorial information (120) reminding the patient to maintain their hands (118) on the electrodes (117) during the ECG recording, and to complete the patient questionnaire (115) following completion of the ECG recording.

6. The device of any preceding claim, wherein the circuit card (124) is programmed for a particular patient in accordance with a particular medication prescribed to the patient.

7. The device of any preceding claim, wherein the source of power is a battery (125) and wherein the circuit card (124) further includes leads (129, 130) connected to the electrodes (117), a user interface (128) and an acquisition interface (131), all interconnected with the processor (126) and the memory (127).

8. A method of limited term monitoring of the patient for cardiac problems, the method comprising the steps of:
(a) providing a portable electrocardiogram (ECG) device (100) as claimed in claim 1;
(b) indicating a start status to the patient when the device (100) is in the unfolded, operative position by means of the status indicator light (119) to signify an ECG recording is required;
(c) touching the electrodes (119) with hands (118) of the patient;
(d) collecting and storing the ECG signal when the patient touches the electrodes (117);
(e) indicating a finished status to the patient by means of the status indicator light (119) to signify the ECG signal has been recorded; and
(f) transferring the stored ECG signal to a remote location for analysis of the stored ECG signal.

9. A method of limited term monitoring of a patient as in claim 8, wherein the monitoring of the patient for cardiac problems is associated with taking of a particular prescribed medication by the patient; wherein the step of providing the patient with a portable personalized electrocardiogram (ECG) device (106) is performed at the time the prescribed medication is dispensed; and wherein the circuit card (124) is programmed in accordance with the particular prescribed medication.

10. The method of claim 9, wherein the step of transferring the stored ECG data includes providing the cover (102) with preprinted mailing information (105) for returning the device (100) in the closed position to the remote location.

## Patentansprüche

1. Portable Elektrokardiogramm-Aufzeichnungseinrichtung (100) mit begrenzter Verwendung zum Diagnostizieren von kardiologischen Problemen, wobei die Einrichtung (100) zwischen einer gefalteten, geschlossenen Position und einer ungefalteten, ein EKHG aufzeichnenden Position verstellbar ist, wobei die Einrichtung (100) aufweist:
eine Abdeckung (102), die klappbar mit einer Basis (104) verbunden ist, wobei die Abdeckung (102) und die Basis (104) jede eine innere Fläche (110, 111) aufweisen, die im Betriebszustand zu einem Patienten hin ausgerichtet ist, wobei die innere Fläche (110) der Abdeckung (104) mit Bedienanweisungen (116) und einem Patientenfragebogen (115) ausgestattet ist, wobei die innere Fläche (111) der Basis (104) mit einem Paar voneinander beabstandeter taktiler sensorischer Elektroden (117) ausgestattet ist, die mit der Patientenhand (118) während einer EKG-Aufzeichnung in Eingriff bringbar sind, und mindestens einem Anzeige-Leuchte (119) zum Signalisieren eines Anfangs- und eines Ende-Status der EKG-Aufzeichnung; und
Mittel (107, 137), die die gespeicherten EKG-Signale an einen entfernten Ort zur Analyse hiervon übertragen;
**dadurch gekennzeichnet, dass**
eine Chipkarte (124) innerhalb eines Schlitzes (123) in der Basis (104) entfernbar aufgenommen wird, wobei die Chipkarte eingerichtet ist, um aus dem Schlitz (123) entfernt zu werden, um die EKG-Daten des Patienten zu analysieren; wobei die Chipkarte (124) elektrisch mit den Elektroden (117) und der Status-Anzeige-Leuchte (119) verbunden ist, wobei die Chipkarte (124) eine Leistungsquelle (125), einen Prozessor (126) und einen Speicher (126) zum Empfangen und Speichern von EKG-Signalen aus dem Patienten enthält, der die Elektroden (117) berührt.

2. Einrichtung gemäß Anspruch 1, worin die Abdeckung (102) eine äußere Fläche (105) aufweist, die mit vorgedruckten Versende-Information (107) versehen ist, zum Zurücksenden der Aufzeichnungseinrichtung (100) in der gefalteten, geschlossene Position an den Ort zur rückwirkenden Analyse der gespeicherten EKG-Signale.

3. Einrichtung gemäß Anspruch 1 oder 2, worin die innere Fläche (110) der Abdeckung (102) bildhafte Bedienanweisungen (112) enthält, die für jeden Patienten allgemein verständlich sind.

4. Einrichtung gemäß einem der vorhergehenden Ansprüche, worin die innere Fläche (110) der Abdeckung (102) ferner eine Halterung (113) zum Aufnehmen und Behalten einer ersten Karte (116), die in jeder gewünschten Sprache gedruckte genaue Text-Bedienanweisungen enthält, und einer zweiten Karte (115) zum Bereitstellen von Gesundheits- und demographischen Informationen von dem Patienten enthält.

5. Einrichtung gemäß einem der vorstehenden Ansprüche, worin die innere Fläche (111) der Basis (104) mit bebilderten Informationen versehen ist, die den Patienten erinnern, seine Hände (118) während der EKG-Aufzeichnung auf den Elektroden (117) zu halten, und den Patienten-Fragebogen (115) nachfolgend nach dem Abschluss der EKG-Aufzeichnung zu beantworten.

6. Einrichtung nach einem der vorhergehenden Ansprüche, worin die Chipkarte (124) für einen bestimmten Patienten entsprechend einer vorgeschriebenen Medikation programmiert ist, die dem Patienten verordnet ist.

7. Einrichtung gemäß einem der vorhergehenden Ansprüche, worin die Leistungsquelle eine Batterie (125) ist und worin die Chipkarte (124) ferner Leitungen (129, 130) enthält, die mit Elektroden (117) verbunden sind, eine Benutzerschnittstelle (128) und eine Akquisitionsschnittstelle (131) vorgesehen sind, wobei alle mit dem Prozessor (126) und dem Speicher (127) verbunden sind.

8. Verfahren für eine Überwachung des Patienten mit Herzproblemen für eine begrenzte Dauer, wobei das Verfahren die Schritte aufweist:
(a) Bereitstellen einer portablen Elektrokardiogramm (EKG)-Einrichtung (100) wie in Anspruch 1 beansprucht;
(b) Anzeigen eines Start-Status für den Patienten mittels der Status-Anzeige-Leuchte (119), wenn die Einrichtung (100) in der ungefalteten Betriebsposition ist, um anzuzeigen, dass eine EKG-Aufzeichnung angefordert wird;
(c) Berühren der Elektroden (119) mit den Händen (118) des Patienten;
(d) Aufnehmen und Speichern des EKG-Signals, wenn der Patient die Elektroden (117) berührt;
(e) Anzeigen eines Beendigungs-Status für den Patienten mittels der Status-Anzeigelicht (119), um anzuzeigen, dass das EKG-Signal aufgezeichnet ist; und
(f) Übertragen des gespeicherten EKG-Signals an einen entfernten Ort zur Analyse des gespeicherten EKG-Signals.

9. Verfahren der Überwachung eines Patienten für eine begrenzte Dauer gemäß Anspruch 8, worin die Überwachung des Patienten mit Herzproblemen mit dem Nehmen einer bestimmten verordneten Medikation durch den Patienten verknüpft ist; worin der Schritt der Ausstattung des Patienten mit der portablen personalisierten Elektrokardiogramm-Einrichtung (106) zu der zeit durchgeführt wird, zu der die vorgeschriebene Medikation verabreicht ist; und worin die Chipkarte (124) gemäß der bestimmten vorgeschriebenen Medikation programmiert ist.

10. Verfahren gemäß Anspruch 9, worin der Schritt der Übertragung der gespeicherten EKG-Daten die Bereitstellung der Abdeckung (102) mit der vorgedruckten Versende-Information (105) zum Zurücksenden der Einrichtung (100) in der geschlossenen Position an den entfernten Ort enthält.

## Revendications

1. Dispositif (100) d'enregistrement d'électrocardiogramme (ECG) portatif, à usage limité, destiné à diagnostiquer des problèmes cardiaques, le dispositif (100) pouvant être déplacé entre une position fermée repliée, a une position de fonctionnement d'enregistrement d'ECG dépliée, le dispositif (100) comprenant :
un couvercle (102) relié de manière articulée à une base (104), le couvercle (102) et la base (104) possédant chacun une face intérieure (110, 111) exposée à un patient lorsqu'ils sont en état de fonctionnement, la face intérieure (110) du couvercle (104) comportant des instructions d'utilisation (116) et un questionnaire (115) sur le patient, la face intérieure (111) de la base (104) comportant une paire d'électrodes espacées (117) à détection tactile, pouvant entrer en contact avec les mains (118) du patient pendant l'enregistrement d'un ECG, et au moins un voyant (119) destiné à signaler un état de début et de fin d'enregistrement de l'électrocardiogramme ; et
des moyens (107, 137) de transfert des signaux ECG stockés vers un emplacement distant en vue de leur analyse, **caractérisé par**
une carte (124) de circuit maintenue de façon amovible à l'intérieur d'une fente (123) dans la base (104), la carte de circuit étant conçue pour être retirée de la fente (123) afin d'analyser les données de l'ECG du patient ; la carte (124) de circuit étant reliée électriquement aux électrodes (117) et au voyant (119) indicateur d'état, la carte (124) de circuit comprenant une source d'alimentation (125), un processeur (126) et une mémoire (127) destinée à recevoir et à stocker des signaux ECG après que le patient ait touché les électrodes (117).

2. Dispositif selon la revendication 1, dans lequel le couvercle (102) possède une face extérieure (105) comportant une information (107) de publipostage pré-imprimée destinées à renvoyer le dispositif (100) d'enregistrement dans la position fermée repliée vers l'emplacement afin d'analyser rétroactivement les signaux ECG stockés.

3. Dispositif selon la revendication 1 ou 2, dans lequel la face intérieure (110) du couvercle (102) comprend des instructions (112) d'utilisation illustrées qui sont compréhensibles de manière universelle par tous les patients.

4. Dispositif selon l'une des revendications précédentes, dans lequel la face intérieure (110) du couvercle (102) comprend en outre un support (113) destiné à recevoir et à retenir une première carte (116) comportant des instructions d'utilisation textuelles détaillées, imprimées dans une langue souhaitée, et une deuxième carte (115) destinée à fournir des informations de santé et démographiques sur le patient.

5. Dispositif selon l'une des revendications précédentes, dans lequel la face intérieure (111) de la base (104) comporte des informations (120) illustrées rappelant au patient de garder ses mains (118) sur les électrodes (117) pendant l'enregistrement de l'électrocardiogramme, et de remplir le questionnaire (115) sur le patient une fois l'enregistrement de l'électrocardiogramme terminé.

6. Dispositif selon l'une des revendications précédentes, dans lequel la carte (124) de circuit est programmée pour un patient spécifique conformément à une médication spécifique prescrite au patient.

7. Dispositif selon l'une des revendications précédentes, dans lequel la source d'alimentation est une batterie (125) et dans lequel la carte (124) de circuit comprend en outre des conducteurs (129, 130) reliés aux électrodes (117), une interface utilisateur (128) et une interface (131) d'acquisition, le tout étant raccordé au processeur (126) et à la mémoire (127).

8. Procédé de surveillance des problèmes cardiaques du patient pour une durée limitée, le procédé comportant les étapes suivantes :
(a) fournir un dispositif (100) d'électrocardiogramme (ECG) portatif selon la revendication 1 ;
(b) indiquer un état de départ au patient lorsque le dispositif (100) est dans la position de fonctionnement dépliée, au moyen du voyant (119) indicateur d'état pour signifier qu'un enregistrement d'électrocardiogramme est nécessaire ;
(c) toucher les électrodes (119) avec les mains (118) du patient ;
(d) collecter et stocker le signal ECG lorsque le patient touche les électrodes (117) ;
(e) indiquer un état fini au patient au moyen du voyant (119) indicateur d'état pour signifier que le signal ECG a été enregistré ; et
(f) transférer le signal ECG stocké à un emplacement distant en vue d'analyser le signal ECG stocké.

9. Procédé de surveillance du patient pour une durée limitée selon la revendication 8, dans lequel la surveillance des problèmes cardiaques du patient est associée à la prise par le patient d'une médication prescrite spécifique ; dans lequel l'étape de fourniture au patient d'un dispositif (106) d'électrocardiogramme (ECG) personnalisé portatif est effectuée au moment où la médication prescrite est dispensée ; et dans lequel la carte (124) de circuit est programmée conformément à la médication prescrite spécifique.

10. Procédé selon la revendication 9, dans lequel l'étape de transfert des données ECG stockées consiste à fournir le couvercle (102) avec une information (105) de publipostage pré-imprimée destinée à renvoyer le dispositif (100) dans la position fermée à l'emplacement distant.
